# FASCICULE DE BREVET EUROPEEN

(11) **EP 2 729 409 B1**
(45) Date de publication et mention de la délivrance du brevet: **07.11.2018**
(21) Numéro de dépôt: 12731567.9
(22) Date de dépôt: 14.06.2012
(51) Int. Cl.: C07C 17/25, C07C 17/38, C07C 21/18, C01B 7/19

(54) **PROCÉDÉ DE SÉPARATION ET RÉCUPÉRATION DU 2,3,3,3-TÉTRAFLUOROPROPÈNE ET DE L'ACIDE FLUORHYDRIQUE**
VERFAHREN ZUM TRENNEN UND WIEDERGEWINNEN VON 2,3,3,3-TETRAFLUORPROPEN UND FLUSSSÄURE
METHOD FOR SEPARATING AND RECOVERING 2,3,3,3-TETRAFLUOROPROPENE AND HYDROFLUORIC ACID

(30) Priorité: 08.07.2011 FR 1156208
(43) Date de publication de la demande: 14.05.2014
(73) Titulaire: Arkema France, 92700 Colombes (FR)
(72) Inventeur: DEUR-BERT, Dominique, 69390 Charly (FR); COLLIER, Bertrand, 69230 Saint-genis-laval (FR)
(74) Mandataire: Dang, Doris
(86) Numéro de dépôt international: PCT/FR2012/051327
(87) Numéro de publication internationale: WO 2013/007906

(56) Documents cités:
- WO-A1-2009/105512
- WO-A2-2008/008519

## Description

La présente invention concerne un procédé de séparation d'une composition renfermant du 2,3,3,3-tetrafluoropropène et de l'acide fluorhydrique et de récupération du 2,3,3,3-tetrafluoropropène et de l'acide fluorhydrique ainsi séparés.

Le choix d'un fluide de transfert de chaleur est dicté d'une part par les propriétés thermodynamiques du fluide, et d'autre part par des contraintes supplémentaires. Ainsi, un critère particulièrement important est celui de l'impact du fluide considéré sur l'environnement.

Les problèmes posés par les substances appauvrissant la couche d'ozone atmosphérique ont été traités à Montréal où a été signé le protocole imposant une réduction de la production et de l'utilisation des chlorofluorocarbures (CFC). Ce protocole a fait l'objet d'amendements qui ont imposé l'abandon des CFC et étendu la réglementation à d'autres produits, dont les hydrochlorofluorocarbones (HCFC).

L'industrie de la réfrigération et de la climatisation a beaucoup investi dans la substitution de ces fluides frigorigènes et c'est ainsi que les hydrofluorocarbures (HFC) ont été commercialisés.

Dans l'industrie automobile, les systèmes de climatisation des véhicules commercialisés dans de nombreux pays sont passés d'un fluide frigorigène au chlorofluorocarbure (CFC-12) à celui de l'hydrofluorocarbure (1,1,1,2-tetrafluoroéthane : HFC-134a), moins nocif pour la couche d'ozone. Cependant, au regard des objectifs fixés par le protocole de Kyoto, le HFC-134a (GWP = 1430) est considéré comme ayant un pouvoir de réchauffement élevé. La contribution à l'effet de serre d'un fluide est quantifiée par un critère, le GWP (Global Warming Potential) qui résume le pouvoir de réchauffement en prenant une valeur de référence de 1 pour le dioxyde de carbone.

Le 2,3,3,3-tetrafluoropropène (HFO-1234yf), du fait de son faible GWP, est considéré comme un candidat potentiel au remplacement du HFC-134a dans la climatisation automobile.

Le 2,3,3,3-tetrafluoropropène est en général préparé en faisant réagir un composé hydrocarbure chloré ou fluorochloré en présence d'une quantité surstoechiométrique d'acide fluorhydrique (WO 2008/054781 ; WO 2008/040969). Ainsi, le flux à l'issue de cette réaction contient non seulement le HFO-1234yf mais aussi de l'HF en quantité non négligeable.

Le document WO 2011/059078 propose de récupérer du HFO-1234yf du mélange à la sortie de la réaction d'hydrofluoration par refroidissement dudit mélange pour obtenir une phase supérieure concentrée en HF et une phase inférieure concentrée en HFO-1234yf, puis par distillation azéotropique de la phase inférieure.

Ce document ne s'intéresse qu'à la purification du HFO-1234yf. Or, on constate que la partie supérieure concentrée en HF contient une quantité importante de HFO-1234yf. Par conséquent, cette partie supérieure doit également être soumise à un traitement si l'on souhaite récupérer de l'HF pour être réutilisé dans la réaction d'hydrofluoration sans recycler inutilement du HFO-1234yf.

Par ailleurs, le document WO 2008/008519 décrit à l'exemple 5, la séparation d'une composition azéotropique de HFO-1234yf et HF par extraction liquide-liquide à l'aide d'un isomère du 1,2,3,3,3-pentafluoropropène à une température de -40°C. Le composé utilisé pour l'extraction est très toxique. Le document WO 2009/105512 décrit un procédé de séparation de mélanges contenant du HF et du HFO-1234yf en présence d'entraîneurs.

La demanderesse a maintenant mis au point un procédé de séparation d'une composition comprenant du 2,3,3,3-tetrafluoropropène et d'HF, et de récupération non seulement du 2,3,3,3-tetrafluoropropène mais aussi de l'HF. Ce procédé ne présente pas les inconvénients de l'art antérieur.

La présente invention a donc pour premier objet un procédé de séparation et récupération du 2,3,3,3-tetrafluoropropène et d'HF à partir d'une composition comprenant du 2,3,3,3-tetrafluoropropène et d'HF caractérisé en ce qu'il comprend une étape de refroidissement de ladite composition en présence d'une quantité ajoutée d'au moins un composé (C1) pour donner une phase supérieure riche en HF et une phase organique inférieure riche en HFO-1234yf et composé C1 ; dans lequel la température de refroidissement est comprise entre -20 et 40°C et de préférence entre -5 et 35°C et dans lequel le composé C1 est choisi parmi le 1,1,1,2,3-pentachloropropane, le 1,1,2,3-tetrachloro-1-fluoropropane, le 1,2-dichloro-3,3,3-trifluoropropane, le 1,1,2,3-tetrachloropropène, le 1,1,1,2-tetrachloropropène et le 2-chloro-3,3,3-trifluoropropène.

Cette étape de refroidissement en présence d'au moins un composé C1 permet d'obtenir une phase supérieure plus riche en HF avec de très faible quantité en HFO-1234yf, susceptible d'être utilisée sans aucune étape de purification. L'HF ainsi récupéré peut être directement recyclé à une étape réactionnelle d'hydrofluoration.

La phase organique inférieure comprend le composé C1, du HFO-1234yf et éventuellement des impuretés organiques. Cette phase organique peut être soumise à une étape de distillation pour séparer le composé C1 et le HFO-1234yf. Le composé C1 peut être recyclé à l'étape de refroidissement et/ou à l'étape réactionnelle conduisant à la formation du HFO-1234yf.

Le ratio molaire du HFO-1234yf/HF dans la composition à séparer est de préférence compris entre 0,5 et 2,5 et avantageusement compris entre 1,1 et 2,1 .

Le 2,3,3,3-tetrafluoropropène est de préférence présent en quantité azéotropique ou quasi-azéotropique avec de l'HF dans la composition à séparer.

Avantageusement la composition à séparer provient d'une étape d'hydrofluoration ou de déhydrofluoration.

De préférence, le composé à ajouter est le même que celui qui a réagi avec de l'HF pour donner le HFO-1234yf ou le composé à ajouter est un intermédiaire de la réaction d'hydrofluoration conduisant à la fabrication du HFO-1234yf.

Par exemple, lorsque le HFO-1234yf est préparé par la réaction d'hydrofluoration du HCC-240db, le composé C1 est de préférence le HCC-240db ou le HCFO-1233xf.

De même, lorsque le HFO-1234yf est préparé par la réaction d'hydrofluoration du HFO-1233xf, le composé C1 est de préférence le HFO-1233xf.

Lorsque le HFO-1234yf est préparé par la réaction d'hydrofluoration du HFO-1230xa, le composé C1 est de préférence le HFO-1230xa ou le HCFO-1233xf.

La quantité de composé C1 à ajouter peut représenter 5 à 95% en poids par rapport au mélange HFO-1234yf - HF et de préférence 10 à 80 % en poids par rapport au mélange HFO-1234yf - HF.

La composition à séparer est de préférence refroidie à une température comprise entre -20 et 40°C et avantageusement à une température comprise entre -5 et 35 °C. La température de refroidissement dépend à la fois de la nature et de la quantité du composé C1 à ajouter. Ainsi, dans le cas d'ajout d'une faible quantité de HCC-240db, la température de l'étape de refroidissement est de préférence voisine de 0°C alors qu'elle peut atteindre la température ambiante (c-à-d 25°C) en présence d'une quantité plus importante d'ajout du composé C1.

La pression à laquelle cette étape de refroidissement est mise en oeuvre est comprise entre 0 à 40 bar de préférence comprise entre 0,3 et 25 bar et avantageusement voisine de celle de l'étape réactionnelle.

Outre le HFO-1234yf et l'HF, la composition à séparer peut comprendre des impuretés organiques telles que le HCFC-243db, le HCFO-1233xf, le 1,1,1,2,2-pentafluoropropane (HFC-245cb), le 1,3,3,3-tetrafluoropropène (HFO-1234ze), le 1-chloro-3,3,3-trifluoropropène (HFO-1233zd) et le 3,3,3-trifluoropropène (HFO-1243zf).

Ces impuretés sont en général des sous-produits de l'étape réactionnelle.

La présente invention a aussi pour objet un procédé de purification du HFO-1234yf. Après l'étape de refroidissement, le HFO-1234yf est séparé du composé C1 de la phase organique, par exemple par distillation. Le composé C1 peut ensuite être recyclé à l'étape de refroidissement et/ou à l'étape réactionnelle lorsque le composé C1 est un réactif ou un intermédiaire de l'étape réactionnelle conduisant à la formation du HFO-1234yf.

Le HFO-1234yf ainsi séparé, peut ensuite être soumis à une étape de purification pour éliminer les impuretés et éventuellement des traces d'HF.

Ainsi, lorsque le HFO-1234yf est séparé par distillation de la phase organique comprenant C1, l'effluent gazeux comprenant majoritairement le HFO-1234yf issu de la colonne de distillation peut être soumis à un lavage dans des colonnes de lavage à l'eau puis à l'eau sodée pour éliminer toute trace d'acidité. Le HFO-1234yf débarrassé de son acidité peut ensuite être séché, comprimé et liquéfié et enfin purifié à l'aide de plusieurs colonnes de distillation, permettant d'éliminer les impuretés légères ou lourdes provenant des étapes précédentes.

L'intérêt de l'étape de refroidissement (décantation) selon la présente invention est en partie de maximiser la récupération d'HF et de minimiser la quantité d'HF contenue dans la phase organique, ce qui se traduit par une minimisation des effluents contenant de l'HF. Elle présente aussi l'intérêt de minimiser la quantité de HFO-1234yf recyclée à l'étape réactionnelle dans la phase enrichie en HF. Par ailleurs, cette décantation peut être mise en oeuvre à température ambiante ce qui permet d'éviter de refroidir le mélange à des températures très basses et d'utiliser un groupe froid, ce qui présente un avantage énergétique certain.

La présente invention a en outre, pour objet, un procédé de fabrication du HFO-1234yf comprenant (i) au moins une étape au cours de laquelle au moins un composé hydrocarboné saturé ou insaturé à trois atomes de carbone comprenant au moins un atome de chlore réagit avec de l'HF en présence d'un catalyseur de fluoration, (ii) une étape d'élimination d'HCl co-produit au cours de l'étape (i), (iii) une étape de refroidissement selon le premier objet de l'invention, et (iv) au moins une étape de purification de HFO-1234yf de la phase organique obtenue en (iii).

De préférence, le composé hydrocarboné saturé ou insaturé à trois atomes de carbone est choisi parmi le HCC-240db, le HCFO-1233xf et le HCO-1230xa.

L'étape de réaction avec de l'HF est de préférence mise en oeuvre en phase gazeuse.

Selon un mode de réalisation préférée de la présente invention, le procédé de fabrication du HFO-1234yf comprend (i) au moins une étape réactionnelle au cours de laquelle du HCC-240db réagit en phase gazeuse avec de l'HF en présence d'un catalyseur de fluoration pour donner un flux comprenant du HFO-1234yf et ses intermédiaires, par exemple le HCFO-1233xf, de l'HCl et de l'HF en excès ; (ii) une étape d'élimination d'HCl du flux sortant de l'étape (i) ; (iii) une étape de refroidissement en présence d'ajout d'un composé C1, de préférence du HCC-240db ou HCFO-1233xf, pour donner une phase supérieure concentrée en HF et une phase inférieure concentrée en HFO-1234yf et comprenant le composé C1 ; (iv) une étape de séparation du HFO-1234yf du composé C1 et éventuellement recyclage du composé C1 à l'étape de refroidissement (iii) ou à l'étape (i) ; (v) une étape de purification du HFO-1234yf et (vi) éventuellement recyclage de la phase supérieure riche en HF à l'étape (i).

En présence d'un large excès d'HF mis en jeu dans l'étape réactionnelle, le procédé de fabrication du HFO-1234yf selon la présente invention peut comprendre, avant l'étape d'élimination d'HCl, une étape de distillation pour récupérer une partie d'HF qui peut être recyclée à l'étape réactionnelle.

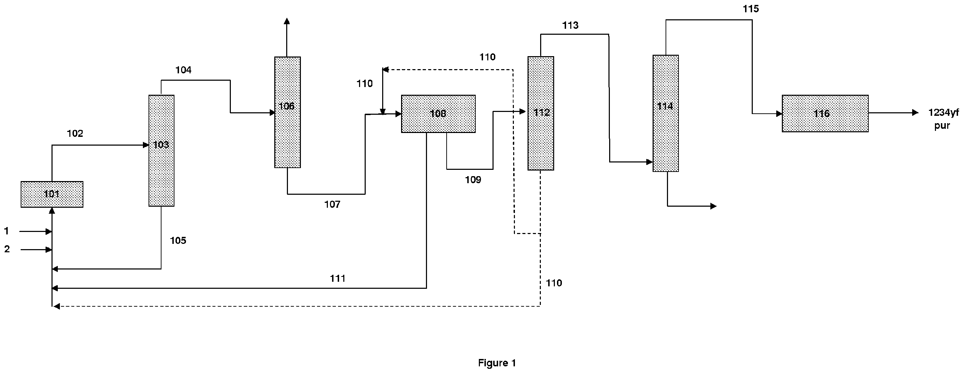

La figure 1 décrite ci-après représente un mode de réalisation préféré de la présente invention.

Du HCC-240db (1) et de l'HF (2), éventuellement préchauffés, sont introduits dans le réacteur (101) contenant un catalyseur de fluoration maintenu à une température comprise entre 300 et 450°C. Le flux (102) à la sortie du réacteur comprenant du HCl, de l'HF, du HFO-1234yf, du HCFO-1233xf et du HFC-245cb est envoyé dans une première colonne de distillation de l'HF (103) pour donner en tête de colonne un flux (104) comprenant de l'HCl, du HFO-1234yf, de l'HF et du HFC-245cb et en pied de colonne un flux (105) comprenant de l'HF, du HCFO-1233xf et du HFC-245cb.

Le flux (104) est envoyé dans une deuxième colonne de distillation (106) pour donner l'HCl en tête et un flux (107) en pied comprenant de l'HF, du HFO-1234yf et du HFC-245cb.

Le flux (105) est recyclé au réacteur (101).

Le décanteur (108) maintenu à une température comprise entre -5 et 35°C est alimenté par le flux (107) et par le flux (110) comprenant du HCC-240db.

La phase inférieure (109) du décanteur comprenant du HFO-1234yf et du HCC-240db est envoyé dans une colonne de distillation (112) pour donner en tête un flux comprenant essentiellement du HFO-1234yf (113) et en pied un flux (110) comprenant essentiellement du HCC-240db.

Le flux (110) est recyclé au réacteur (101) et/ou au décanteur (108).

La phase supérieure (111) du décanteur enrichie en HF est recyclée au réacteur (101).

Le flux (113) est envoyé dans les colonnes de lavage (114) pour donner en tête un flux (115) débarrassé de toute acidité.

Le flux (115) est enfin envoyé à l'étape de purification (116) pour donner du HFO-1234yf pur.

### PARTIE EXPERIMENTALE

Une composition liquide contenant 40 % molaire de HF et 60 % mol de HFO-1234yf (soit 89,5% en poids) est refroidie à pression atmosphérique à une température légèrement supérieure à 15°C. Il n'y a pas de décantation.

A une température inférieure à 15°C, on obtient une phase organique inférieure contenant plus de 92 % en poids de HFO-1234yf et une phase supérieure contenant seulement 71% en poids de HF.

A -30°C, la phase supérieure contient seulement 78 % en poids de HF et 22% en poids de HFO-1234yf ce qui reste très important. La phase organique inférieure contient 98,5% en poids HFO-1234yf et 1,5% en poids d'HF. L'efficacité de la décantation est donc médiocre même à basse température.

### Exemples selon l'invention

### Exemple 1

Une composition liquide contenant 32,6 % en poids de HFO-1234yf, 3,9 % en poids d'HF (ratio molaire HFO-1234yf/HF = 1,48) et 63,5 % en poids de HCC-240db est refroidie.

A une température de refroidissement de 20°C, on obtient une phase organique inférieure contenant 0,2 % en poids d'HF et une phase supérieure contenant 88% en poids d'HF et 10 % en poids de HFO-1234yf.

A une température de refroidissement de 0°C, on obtient une phase organique inférieure contenant 0,1 % en poids d'HF et une phase supérieure contenant 89% en poids d'HF et 9 % en poids de HFO-1234yf.

### Exemple 2

Une composition liquide contenant 36,5 % en poids de HFO-1234yf, 4,3% en poids d'HF (ratio molaire HFO-1234yf/HF = 1.5) et 59,2 % en poids de HCC-1230xa est refroidie.

A une température de refroidissement de 20°C, on obtient une phase organique inférieure contenant 0,2 % en poids d'HF et une phase supérieure contenant 86 % en poids d'HF et 11 % en poids de HFO-1234yf.

A une température de refroidissement de 0°C, on obtient une phase organique inférieure contenant 0,15 % en poids d'HF et une phase supérieure contenant 87 % en poids d'HF et 11 % en poids de HFO-1234yf.

### Exemple 3

Une composition liquide contenant 38,1 % en poids de HFO-1234yf, 4,5 % en poids d'HF et 57,4 % en poids de HCFC-243db est refroidie.
A une température de refroidissement de 20°C, on obtient une phase organique inférieure contenant 0,1 % en poids d'HF et une phase supérieure contenant 85 % en poids d'HF et 9 % en poids de HFO-1234yf.

A une température de refroidissement de -20°C, on obtient une phase organique inférieure contenant moins de 0,1 % en poids d'HF et une phase supérieure contenant 88 % en poids d'HF et 9 % en poids de HFO-1234yf.

### Exemple 4

Une composition liquide contenant 43,6 % en poids de HFO-1234yf, 5,2 % en poids d'HF et 51,2 % en poids de HCFC-1233xf est refroidie.

A une température de refroidissement de 0°C, on obtient une phase organique inférieure contenant 2 % en poids d'HF et une phase supérieure contenant 77% en poids d'HF et 11 % en poids de HFO-1234yf.

### Exemple 5

Une composition liquide contenant 40,4 % en poids de HFO-1234yf, 4,8 % en poids d'HF et 54,8 % en poids de HCFC-244bb est refroidie.

A une température de refroidissement de 20°C, on obtient une phase organique inférieure contenant 1,5 % en poids d'HF et une phase supérieure contenant 82% en poids d'HF et 10 % en poids de HFO-1234yf.

A une température de refroidissement de 0°C, on obtient une phase organique inférieure contenant 1 % en poids d'HF et une phase supérieure contenant 84% en poids d'HF et 9 % en poids de HFO-1234yf.

## Revendications

1. Procédé de séparation et récupération du 2,3,3,3-tetrafluoropropène et d'HF à partir d'une composition comprenant du 2,3,3,3-tetrafluoropropène et d'HF **caractérisé en ce qu'**il comprend une étape de refroidissement de ladite composition en présence d'une quantité ajoutée d'au moins un composé (C1) pour donner une phase supérieure riche en HF et une phase organique inférieure riche en HFO-1234yf et composé C1 ; dans lequel la température de refroidissement est comprise entre -20 et 40°C et de préférence entre -5 et 35°C et dans lequel le composé C1 est choisi parmi le 1,1,1,2,3-pentachloropropane, le 1,1,2,3-tetrachloro-1-fluoropropane, le 1,2-dichloro-3,3,3-trifluoropropane, le 1,1,2,3-tetrachloropropène, le 1,1,1,2-tetrachloropropène et le 2-chloro-3,3,3-trifluoropropène.

2. Procédé selon la revendication 1 **caractérisé en ce que** le ratio molaire du 2,3,3,3-tetrafluoropropène /d'HF est compris entre 0,5 et 2,5, de préférence compris entre 1,1 et 2,1.

3. Procédé selon la revendication 1 ou 2 **caractérisé en ce que** a quantité de composé C1 à ajouter représente 5 à 95% en poids par rapport au mélange HFO-1234yf - HF, de préférence 10 à 80 % en poids par rapport au mélange HFO-1234yf - HF.

4. Procédé selon l'une quelconque des revendications précédentes **caractérisé en ce que** la composition comprenant du 2,3,3,3-tetrafluoropropène et d'HF provient d'une réaction d'hydrofluoration d'au moins un composé hydrocarboné saturé ou insaturé à trois atomes de carbone comprenant au moins un atome de chlore en présence d'un catalyseur de fluoration.

5. Procédé selon la revendication 4 **caractérisé en ce que** le composé hydrocarboné saturé ou insaturé à trois atomes de carbone est choisi parmi le 1,1,1,2,3-pentachloropropane, le 1,1,2,3-tetrachloropropène et le 2-chloro-3,3,3-trifluoropropène.

6. Procédé selon la revendication 4 ou 5 **caractérisé en ce que** le flux issu de l'étape d'hydrofluoration est soumis à une étape d'élimination de HCl avant l'étape de refroidissement.

7. Procédé selon l'une quelconque des revendications 4 à 6 **caractérisé en ce que** le composé C1 est le même que celui qui a réagi avec de l'HF dans l'étape d'hydrofluoration.

8. Procédé selon l'une quelconque des revendications 4 à 7 **caractérisé en ce que** la partie enrichie en HF après l'étape de refroidissement est recyclée à l'étape d'hydrofluoration.

9. Procédé selon l'une quelconque des revendications précédentes **caractérisé en ce que** le 2,3,3,3-tetrafluoropropène est séparé de la partie organique enrichie en 2,3,3,3-tetrafluoropropène après l'étape de refroidissement.

10. Procédé selon la revendication 9 **caractérisé en ce que** la partie organique, après séparation du 2,3,3,3-tetrafluoropropène est recyclée à l'étape de refroidissement et/ou à l'étape réactionnelle.

11. Procédé de fabrication du 2,3,3,3-tetrafluoropropène comprenant (i) au moins une étape réactionnelle au cours de laquelle du 1,1,1,2,3-pentachloropropane réagit en phase gazeuse avec de l'HF en présence d'un catalyseur de fluoration pour donner un flux comprenant du 2,3,3,3-tetrafluoropropène et ses intermédiaires, de l'HCl et de l'HF en excès ; (ii) une étape d'élimination d'HCl du flux sortant de l'étape (i) ; (iii) une étape de refroidissement en présence d'ajout du 1,1,1,2,3-pentachloropropane selon l'une quelconque des revendications 1 à 4 pour donner une phase supérieure riche en HF et une phase organique inférieure riche en 2,3,3,3-tetrafluoropropène et comprenant le 1,1,1,2,3-pentachloropropane; (iv) une étape de séparation du 2,3,3,3-tetrafluoropropène de la phase inférieure et événtuellement recyclage du 1,1,1,2,3-pentachloropropane à l'étape de refroidissement (iii) et/ou à l'étape réactionnelle (i); (v) une étape de purification du 2,3,3,3-tetrafluoropropène et (vi) éventuellement recyclage de la phase supérieure riche en HF à l'étape (i).

12. Procédé selon la revendication 11 **caractérisé en ce qu'**il comprend une étape de distillation d'HF avant l'étape d'élimination d'HCl.

## Patentansprüche

1. Verfahren zur Trennung und Gewinnung von 2,3,3,3-Tetrafluorpropen und HF ausgehend von einer Zusammensetzung, die 2,3,3,3-Tetrafluorpropen und HF umfasst, **dadurch gekennzeichnet, dass** es einen Schritt der Abkühlung der Zusammensetzung in Gegenwart einer zugesetzten Menge mindestens einer Verbindung (C1) zum Erhalt einer an HF reichen oberen Phase und einer an HFO-1234yf und Verbindung C1 reichen unteren Phase; wobei die Abkühlungstemperatur zwischen -20 und 40 °C und vorzugsweise zwischen -5 und 35 °C liegt und wobei die Verbindung C1 aus 1,1,1,2,3-Pentachlorpropan, 1,1,2,3-Tetrachlor-1-fluorpropan, 1,2-Dichlor-3,3,3-trifluorpropan, 1,1,2,3-Tetrachlorpropen, 1,1,1,2-Tetrachlorpropen und 2-Chlor-3,3,3-trifluorpropen ausgewählt wird.

2. Verfahren nach Anspruch 1, **dadurch gekennzeichnet, dass** das 2,3,3,3-Tetrafluorpropen/HF-Molverhältnis zwischen 0,5 und 2,5, vorzugsweise zwischen 1,1 und 2,1, liegt.

3. Verfahren nach Anspruch 1 oder 2, **dadurch gekennzeichnet, dass** die zuzusetzende Menge von Verbindung C1 5 bis 95 Gew.-%, bezogen auf das HFO-1234yf/HF-Gemisch, vorzugsweise 10 bis 80 Gew.-%, bezogen auf das HFO-1234yf/HF-Gemisch, ausmacht.

4. Verfahren nach einem der vorhergehenden Ansprüche, **dadurch gekennzeichnet, dass** die Zusammensetzung, die 2,3,3,3-Tetrafluorpropen und HF umfasst, aus einer Hydrofluorierungsreaktion mindestens einer gesättigten oder ungesättigten Kohlenwasserstoffverbindung mit drei Kohlenstoffatomen und mindestens einem Chloratom in Gegenwart eines Fluorierungskatalysators stammt.

5. Verfahren nach Anspruch 4, **dadurch gekennzeichnet, dass** die gesättigte oder ungesättigte Kohlenwasserstoffverbindung mit drei Kohlenstoffatomen aus 1,1,1,2,3-Pentachlorpropan, 1,1,2,3-Tetrachlorpropen und 2-Chlor-3,3,3-trifluorpropen ausgewählt wird.

6. Verfahren nach Anspruch 4 oder 5, **dadurch gekennzeichnet, dass** der Strom aus dem Hydrofluorierungsschritt vor dem Abkühlungsschritt einem Schritt der Entfernung von HCl unterworfen wird.

7. Verfahren nach einem der Ansprüche 4 bis 6, **dadurch gekennzeichnet, dass** es sich bei der Verbindung C1 um die gleiche Verbindung handelt, die im Hydrofluorierungsschritt mit HF reagiert hat.

8. Verfahren nach einem der Ansprüche 4 bis 7, **dadurch gekennzeichnet, dass** der mit HF angereicherte Teil nach dem Abkühlungsschritt zum Hydrofluorierungsschritt zurückgeführt wird.

9. Verfahren nach einem der vorhergehenden Ansprüche, **dadurch gekennzeichnet, dass** das 2,3,3,3-Tetrafluorpropen nach dem Abkühlungsschritt von dem mit 2,3,3,3-Tetrafluorpropen angereicherten organischen Teil getrennt wird.

10. Verfahren nach Anspruch 9, **dadurch gekennzeichnet, dass** der organische Teil nach Abtrennung des 2,3,3,3-Tetrafluorpropens zum Abkühlungsschritt und/oder zum Reaktionsschritt zurückgeführt wird.

11. Verfahren zur Herstellung von 2,3,3,3-Tetrafluorpropen, umfassend (i) mindestens einen Reaktionsschritt, in dessen Verlauf 1,1,1,2,3-Pentachlorpropan in der Gasphase in Gegenwart eines Fluorierungskatalysators mit HF reagiert, was einen Strom, der 2,3,3,3-Tetrafluorpropen und dessen Zwischenprodukte, HCl und überschüssiges HF umfasst, ergibt; (ii) einen Schritt der Entfernung von HCl aus dem Strom aus Schritt (i); (iii) einen Schritt der Abkühlung in Gegenwart eines Zusatzes von 1,1,1,2,3-Pentachlorpropan nach einem der Ansprüche 1 bis 4 zum Erhalt einer an HF reichen oberen Phase und einer an 2,3,3,3-Tetrafluorpropen reichen und 1,1,1,2,3-Pentachlorpropan umfassenden unteren Phase; (iv) einen Schritt der Abtrennung des 2,3,3,3-Tetrafluorpropens aus der unteren Phase und gegebenenfalls Rückführung des 1,1,1,2,3-Pentachlorpropans zum Abkühlungsschritt (iii) und/oder zum Reaktionsschritt (i); (v) einen Schritt der Reinigung des 2,3,3,3-Tetrafluorpropens und (vi) gegebenenfalls Rückführung der an HF reichen oberen Phase zu Schritt (i).

12. Verfahren nach Anspruch 11, **dadurch gekennzeichnet, dass** es einen Schritt der Destillation von HF vor der Stufe der Entfernung von HCl umfasst.

## Claims

1. Process for the separation and recovery of 2,3,3,3-tetrafluoropropene and HF from a composition comprising 2,3,3,3-tetrafluoropropene and HF, **characterized in that** it comprises a stage of cooling said composition in the presence of an added amount of at least one compound (C1) in order to give an upper phase rich in HF and a lower organic phase rich in HFO-1234yf and compound C1; in which the cooling temperature is between -20 and 40°C and preferably between -5 and 35°C and in which compound C1 is chosen from 1,1,1,2,3-pentachloropropane, 1,1,2,3-tetrachloro-1-fluoropropane, 1,2-dichloro-3,3,3-trifluoropropane, 1,1,2,3-tetrachloropropene, 1,1,1,2-tetrachloropropene and 2-chloro-3,3,3-trifluoropropene.

2. Process according to Claim 1, **characterized in that** the 2,3,3,3-tetrafluoropropene/HF molar ratio is between 0.5 and 2.5, preferably between 1.1 and 2.1.

3. Process according to Claim 1 or 2, **characterized in that** the amount of compound C1 to be added represents from 5% to 95% by weight, with respect to the HFO-1234yf/HF mixture, preferably from 10% to 80% by weight, with respect to the HFO-1234yf/HF mixture.

4. Process according to any one of the preceding claims, **characterized in that** the composition comprising 2,3,3,3-tetrafluoropropene and HF originates from a hydrofluorination reaction on at least one saturated or unsaturated hydrocarbon compound having three carbon atoms and comprising at least one chlorine atom in the presence of a fluorination catalyst.

5. Process according to Claim 4, **characterized in that** the saturated or unsaturated hydrocarbon compound having three carbon atoms is chosen from 1,1,1,2,3-pentachloropropane, 1,1,2,3-tetrachloropropene and 2-chloro-3,3,3-trifluoropropene.

6. Process according to Claim 4 or 5, **characterized in that** the stream resulting from the hydrofluorination stage is subjected to a stage of removal of HCl before the cooling stage.

7. Process according to any one of Claims 4 to 6, **characterized in that** the compound C1 is the same as that which has reacted with HF in the hydrofluorination stage.

8. Process according to any one of Claims 4 to 7, **characterized in that** the part enriched in HF after the cooling stage is recycled to the hydrofluorination stage.

9. Process according to any one of the preceding claims, **characterized in that** the 2,3,3,3-tetrafluoropropene is separated from the organic part enriched in 2,3,3,3-tetrafluoropropene after the cooling stage.

10. Process according to Claim 9, **characterized in that** the organic part, after separation of the 2,3,3,3-tetrafluoropropene is recycled to the cooling stage and/or to the reaction stage.

11. Process for the manufacture of 2,3,3,3-tetrafluoropropene, comprising (i) at least one reaction stage during which 1,1,1,2,3-pentachloropropane reacts in the gas phase with the HF in the presence of a fluorination catalyst to give a stream comprising 2,3,3,3-tetrafluoropropene and its intermediates, HCl and excess HF; (ii) a stage of removal of HCl from the stream exiting from stage (i) ; (iii) a stage of cooling in the presence of the addition of 1,1,1,2,3-pentachloropropane according to any one of Claims 1 to 4 to give an upper phase rich in HF and a lower organic phase rich in 2,3,3,3-tetrafluoropropene and comprising 1,1,1,2,3-pentachloropropane; (iv) a stage of separation of the 2,3,3,3-tetrafluoropropene from the lower phase and optionally recycling the 1,1,1,2,3-pentachloropropane to the cooling stage (iii) and/or to the reaction stage (i); (v) a stage of purification of the 2,3,3,3-tetrafluoropropene and (vi) optionally recycling of the upper phase rich in HF to stage (i).

12. Process according to Claim 11, **characterized in that** it comprises a stage of distillation of HF before the stage of removal of HCl.
